(19) Europäisches Patentamt
European Patent Office
Office européen des brevets

(11) Publication number: 0 404 355 A1

# (12) EUROPEAN PATENT APPLICATION

(21) Application number: 90305635.6

(22) Date of filing: 23.05.90

(51) Int. Cl.[5]: C07D 471/04, A61K 31/505, //(C07D471/04,239:00,221:00)

The title of the invention has been amended (Guidelines for Examination in the EPO, A-III, 7.3).

(30) Priority: 30.05.89 GB 8912336

(43) Date of publication of application: 27.12.90 Bulletin 90/52

(84) Designated Contracting States: CH DE FR GB IT LI NL

(71) Applicant: SMITHKLINE BECKMAN INTERCREDIT B.V.
28-34 Blaak P.O. Box 2
NL-3000 DG Rotterdam(NL)

(72) Inventor: Brown, Thomas Henry, Smith Kline & French Res.Ltd.
The Frythe, Welwyn
Hertfordshire, AL6 9AR(GB)
Inventor: Ife, Robert John, Smith Kline & French Res.Ltd.
The Frythe, Welwyn
Hertfordshire, AL6 9AR(GB)
Inventor: Leach, Colin Andrew, Smith Kline & French Res.Ltd.
The Frythe, Welwyn
Hertfordshire, AL6 9AR(GB)

(74) Representative: Giddings, Peter John, Dr. et al
SmithKline Beecham, Corporate Patents, Mundells
Welwyn Garden City, Hertfordshire AL7 1EY(GB)

## (54) Pyridopyrimidine derivatives, process for the preparation thereof and pharmaceutical compositions containing them.

(57) Compounds of structure

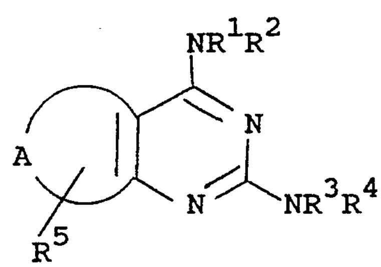

in which A is a pyridyl ring; $R^1$ and $R^2$ are the same, or different and are each hydrogen, $C_{1-4}$alkyl, $-(CH_2)_nAr$ in which n is 0 to 4 and Ar is an optionally substituted phenyl group, or $R^1$ and $R^2$ together with the nitrogen atom to which they are attached form a saturated or unsaturated ring optionally containing one or more further heteroatoms; $R^3$ and $R^4$ are the same or different and are each hydrogen, $C_{1-4}$alkyl, $(CH_2)_nAr^1$ in which n is 0 to 4 and $Ar^1$ is an optionally substituted phenyl group, or $R^3$ and $R^4$ together with the nitrogen atom to which they are attached form a saturated or unsaturated ring optionally containing one or more further heteroatoms; and $R^5$ is hydrogen or $C_{1-4}$alkyl; processes for their preparation, pharmaceutical compositions containing them and their use in therapy as anti-ulcer agents.

EP 0 404 355 A1

## COMPOUNDS

The present invention relates to substituted pyridopyrimidine derivatives, processes for their preparation, intermediates useful in their preparation, pharmaceutical compositions containing them and their use in therapy.

Accordingly the present invention provides, in a first aspect compounds of structure (I)

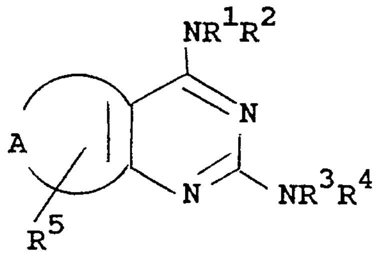

(I)

in which

A is a pyridyl ring;

$R^1$ and $R^2$ are the same, or different and are each hydrogen, $C_{1-4}$alkyl, $-(CH_2)_nAr$ in which n is 0 to 4 and Ar is an optionally substituted phenyl group, or $R^1$ and $R^2$ together with the nitrogen atom to which they are attached form a saturated or unsaturated ring optionally containing one or more further heteroatoms;

$R^3$ and $R^4$ are the same or different and are each hydrogen, $C_{1-4}$alkyl, $(CH_2)_nAr^1$ in which n is O to 4 and $Ar^1$ is an optionally substituted phenyl group, or $R^3$ and $R^4$ together with the nitrogen atom to which they are attached form a saturated or unsaturated ring optionally containing one or more further heteroatoms; and

$R^5$ is hydrogen or $C_{1-4}$alkyl;

and pharmaceutically acceptable salts thereof.

Suitably the pyridyl ring A is attached to the pyrimidine ring so as to form a [3.4-d], [4,3-d] or [3,2-d] system. Preferably, the pyridyl ring is attached so as to form a [2,3-d] system i.e. of structure

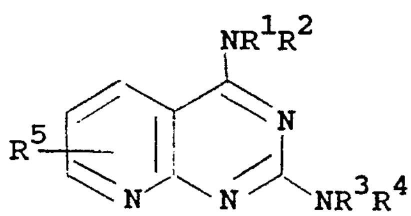

Suitably $R^1$ and $R^2$ are the same or different and are each hydrogen or $(CH_2)_nAr$ in which n is 0 to 4 and Ar is an optionally substituted phenyl group or $R^1$ and $R^2$ together with the nitrogen atom to which they are attached form a saturated or unsaturated ring optionally containing one or more further heteroatoms. More suitably, one of $R^1$ and $R^2$ is hydrogen or $C_{1-4}$alkyl and the other is hydrogen, $C_{1-4}$alkyl or $(CH_2)_nAr$. Most suitably one of $R^1$ and $R^2$ is hydrogen or $C_{1-4}$alkyl and the other is $(CH_2)_nAr$. Preferably one of $R^1$ and $R^2$ is $C_{1-4}$alkyl and the other is $(CH_2)_nAr$; most preferably one of $R^1$ and $R^2$ is $C_{1-4}$alkyl, in particular methyl and the other is $(CH_2)_nAr$ in which n is O.

Suitably, Ar is unsubstituted or substituted by 1 to 3 substituents selected from hydrogen, $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl. More suitably, Ar is unsubstituted or substituted by two substituents selected from hydrogen, $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, in particular $C_{1-4}$alkyl and $C_{1-4}$alkoxy. Preferably, Ar is unsubstituted or substituted by a single substituent selected from the above-noted groups, in particular $C_{1-4}$alkyl or $C_{1-4}$alkoxy. Most preferably Ar is unsubstituted.

Suitably, $R^3$ and $R^4$ are the same or different and are each hydrogen, $C_{1-4}$alkyl, $(CH_2)_nAr^1$ in which n is 0 to 4 and $Ar^1$ is an optionally substituted phenyl group, or $R^3$ and $R^4$ together with the nitrogen atom to

which they are attached form a saturated or unsaturated ring optionally containing one or more further heteroatoms.

More suitably one of $R^3$ and $R^4$ is hydrogen or $C_{1-4}$alkyl and the other is hydrogen, $C_{1-4}$alkyl or $(CH_2)_nAr^1$, or $R^3$ and $R^4$ together with the nitrogen atom to which they are attached form a saturated or unsaturated carbocyclic ring. Most suitably, one of $R^3$ and $R^4$ is hydrogen or $C_{1-4}$alkyl and the other is hydrogen, $C_{1-4}$alkyl or $(CH_2)_nAr^1$. Preferably one of $R^3$ and $R^4$ is hydrogen or $C_{1-4}$alkyl and the other is $(CH_2)_nAr^1$; more preferably one of $R^3$ and $R^4$ is hydrogen and the other is $(CH_2)_nAr^1$; most preferably, one of $R^3$ and $R^4$ is hydrogen and the other is $(CH_2)_nAr^1$ in which n is O.

Suitably, the group $Ar^1$ is unsubstituted or optionally substituted by 1 to 3 substituents as hereinabove described for the group Ar in $R^1$ or $R^2$. Preferably the group $Ar^1$ is unsubstituted or substituted by one or two groups, for example a $C_{1-4}$alkyl group, in particular a methyl group or a halogen atom, in particular a fluorine atom; or a $C_{1-4}$alkyl group and a halogen atom, in particular a methyl group and fluorine atom. More preferably the group $Ar^1$ is substituted by a methyl group in the 2-position of the ring and a fluorine atom in the 4-position of the ring.

Suitably $R^5$ is $C_{1-4}$alkyl; preferably $R^5$ is hydrogen.

$C_{1-4}$alkyl groups (either alone or as part of another group) can be straight or branched.

It will be appreciated that compounds of structure (I) in which one or more of $R^1$ to $R^4$ is a $C_{3-4}$alkyl group (either alone or as part of another group) may contain an assymetric centre due to the presence of the $C_{3-4}$alkyl group. Such compounds will exist as two (or more) optical isomers (enantiomers). Both the pure enantiomers, racemic mixtures (50% of each enantiomer) and unequal mixtures of the two are included within the scope of the present invention. Further, all diastereomeric forms possible (pure enantiomers and mixtures thereof) are within the scope of the invention.

The compounds of the present invention can be prepared by processes analogous to those known in the art. The present invention therefore provides in a further aspect a process for the preparation of a compound of structure (I) or a pharmaceutically acceptable salt thereof which comprises

(a) reaction of a compound of structure (II)

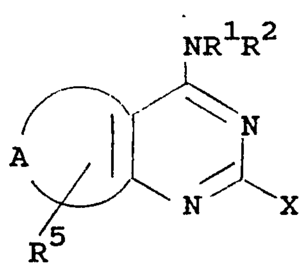

(II)

in which A, $R^1$, $R^2$ and $R^5$ are as described for structure (I) except that where necessary they are in protected form, and X is a group displaceable by an amine, with an amine of structure $R^3R^4NH$ in which $R^3$ and $R^4$ are as described for structure (I);

(b) reaction of a compound of structure (III)

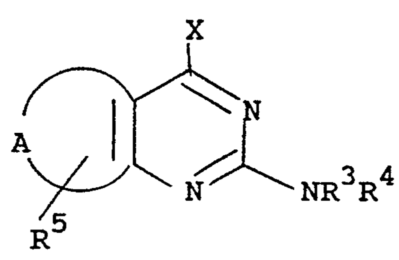

(III)

in which A, $R^3$, $R^4$ and $R^5$ are as described for structure (I) and X is a group displaceable by an amine, with an amine of structure $R^1R^2NH$ in which $R^1$ and $R^2$ are as described for structure (I); or

(c) for compounds in which $NR^1R^2$ and $NR^3R^4$ together are the same, reaction of a compound of structure (IV)

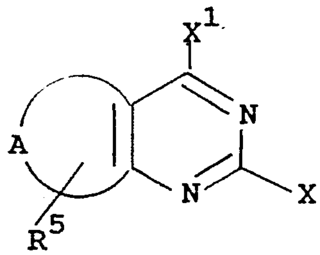

(IV)

in which A and $R^5$ are as described for structure (I) X and $X^1$ are groups displaceable by an amine, with an amine of structure $R^1R^2NH$ or $R^3R^4NH$ in which $R^1$ to $R^4$ are as hereinbefore defined; and optionally thereafter,

° removing any protecting groups;
° forming a pharmaceutically acceptable salt.

Suitable groups displaceable by an amine, X and $X^1$, will be apparent to those skilled in the art and include, for example, halogen, in particular chlorine, $SC_{1-4}$alkyl, such as methylthio, hydroxy and phenoxy.

Reaction of a compound of structure (II) with an amine $R^3R^4NH$ is suitably carried out in an inert solvent at elevated temperature. Preferably the reaction is carried out in the absence of a solvent in a sealed receptacle at elevated temperature.

Reaction of a compound of structure (III) with a amine $R^1R^2NH$ or a compound of structure (IV) with a suitable amine is suitably carried out in the presence or absence of an inert solvent at elevated temperature. Suitable solvents include, for example $C_{1-4}$alkanols such as isopropanol or butanol, preferably isopropanol.

In particular, leaving groups X and $X^1$ are halogen, preferably chlorine, and can be displaced by appropriate amines $R^1R^2NH$ and $R^3R^4NH$ under the general conditions described above and in the specific examples. Other conditions and reagents depending on the nature of the leaving groups will be apparent to those skilled in the art; for example compounds of structure (I) in which $R^5$ and $R^2$ are both hydrogen, can be prepared from the corresponding compounds of structure (III) in which X is hydroxy by reaction with phenylphosphordiamidate using the method described in J. Het. Chem (1972), 9, 1235.

Pharmaceutically acceptable acid addition salts of the compounds of structure (I) can be prepared by standard procedures by, for example, reaction with suitable organic and inorganic acids the nature of which will be apparent to persons skilled in the art. For example, pharmaceutically acceptable salts can be formed by reaction with hydrochloric, sulphuric, or phosphoric acids; aliphatic, aromatic or heterocyclic sulphonic acids or carboxylic acids such as, for example, citric, maleic or fumaric acids.

The intermediate compounds of structure (II), (III) and (IV) can be prepared by procedures analogous to those known in the art. The amines of structure $R^1R^2NH$ and $R^3R^4NH$ are available commercially or can be prepared by standard techniques well known to those skilled in the art of organic chemistry.

For example compounds of structure (II) in which X is chlorine and the A ring is attached so as to form a [2,3-d] ring system can be prepared via compounds of structure (IV) by the route outlined in Scheme I.

Scheme I

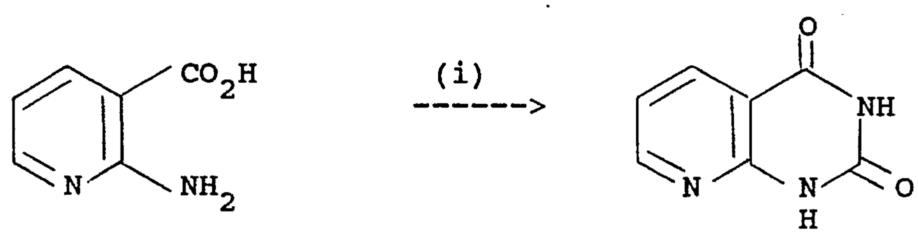


(A) (B)

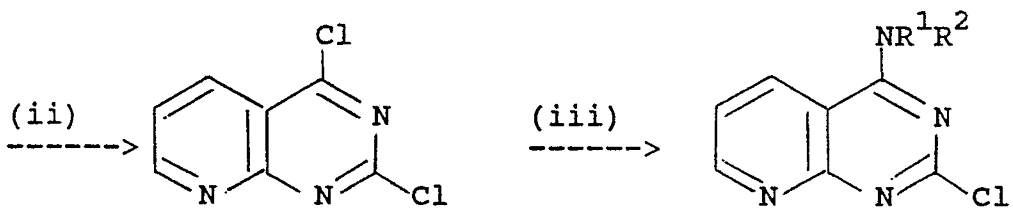


(IV, X = $X^1$ = Cl) (II)

(i) $(H_2N)_2CO$

(ii) $POCl_3$, $PhNMe_2$, Δ

(iii) $R^1R^2NH$, NaOAc, THF/$H_2O$, Δ.

Compounds of structure (III) in which $R^3$ and $R^4$ are both hydrogen, $C_{1-4}$alkyl or $(CH_2)_nAr^1$ or one is $C_{1-4}$alkyl and the other is $(CH_2)_nAr^1$, X is chlorine and the A ring is attached so as to form a [2,3-d] ring system can be prepared by the procedures outlined in Scheme II.

Scheme II

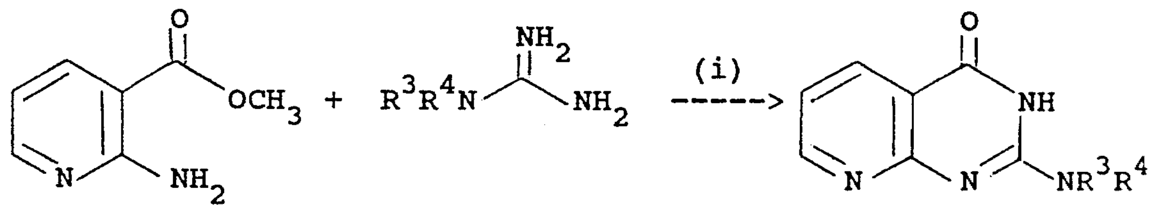


(C) (D) (E)

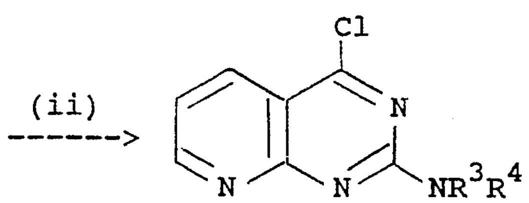


(III)

(i) $NaOCH_3$, nBuOH

(ii) $POCl_3$

The starting materials used to prepare compounds of structures (II) and (III) are available commercially or can be prepared by standard techniques. In addition it will be appreciated that further variations of the above-noted schemes can be utilized to prepare compounds of structure (I) other than those specifically illustrated.

It is to be noted, and apparent to those skilled in the art that in the foregoing reactions, where necessary groups on aromatic rings Ar and $Ar^1$ (e.g. hydroxy or amino groups) will be in "protected" form. For example, amino groups can be "protected" in the form of nitro groups and converted into amino groups as appropriate, and hydroxy groups can be protected using standard groups for example as described in "Greene, T.W., Protective Groups in Organic Chemistry" which also provides examples of further appropriate protective groups for other moities.

The compounds of structure (I) and their pharmaceutically acceptable salts exert an anti-secretory effect by inhibition of the gastrointestinal $H^+K^+$ATPase enzyme (Fellenius E., Berglindh T., Sachs G., Olke L., Elander B., Sjostrand S.E., and Wallmark B., 1981, Nature, 290, 159-61).

In a further aspect therefore the present invention provides compounds of structure (I) and pharmaceutically acceptable salts thereof for use in therapy.

The compounds of structure (I) and their pharmaceutically acceptable salts inhibit exogenously and endogenously stimulated gastric acid secretion and are useful in the treatment of gastrointestinal diseases in mammals, in particular humans. Such diseases include, for example, gastric and duodenal ulcers, and Zollinger-Ellison Syndrome. Further, the compounds of structure (I) can be used in the treatment of other disorders where an anti-secretory effect is desirable for example in patients with gastritis, NSAID induced gastritis, gastric ulcers, acute upper intestinal bleeding, in patients with a history of chronic and excessive alcohol consumption, and in patients with gastro oesophageal reflux disease (GERD).

In addition to the foregoing use the compounds of structure (I) can be expected to be of use in medicine as inhibitors of bone resorption. In normal subjects there is a balance between bone resorption

and bone formation, however in subjects with bone affected diseases such as osteoporosis, Paget's disease and hyperparathyroidism and related disorders this balance is disturbed. As a consequence the subject suffers a loss of bone tissue, decreased bone mass and bone fragility which can result in fracturing of bones. Bone resorption (or bone loss) is associated with the activity of osteoclast cells, and it is thought that agents which inhibit the activity of such cells (and so inhibit bone resorption) will have a beneficial effect on the reduction of bone loss and be of benefit in the treatment of the above-noted disease states. The present compounds can be expected to be inhibitors of osteoclast activity and bone resorption and to be of use in medicine in the treatment of diseases in which bone loss is a factor, in particular osteoporosis, Paget's disease and hyperparathyroidism.

In therapeutic use, the compounds of the present invention are usually administered in a standard pharmaceutical composition. The present invention therefore provides in a further aspect pharmaceutical compositions comprising a compound of structure (I) or a pharmaceutically acceptable salt thereof and a pharmaceutically acceptable carrier.

The compounds of structure (I) and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free base.

The present invention also provides a method of inhibiting gastric acid secretion which comprises administering to a mammal in need thereof an effective amount of a compound of structure (I) or a pharmaceutically acceptable salt thereof; and a method of treatment of diseases of the stomach or intestine based on increased acid secretion which comprises administering to a mammal in need thereof an effective amount of a compound of structure (I) or a pharmaceutically acceptable salt thereof.

The pharmaceutically acceptable compounds of the invention will normally be administered to a subject for the treatment of gastrointestinal diseases and other conditions caused or exacerbated by gastric acidity.

The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

In addition, the compounds of the present invention can be co-administered with further active ingredients, such as antacids (for example magnesium carbonate or hydroxide and aluminium hydroxide), non-steroidal anti-flammatory drugs (for example indomethacin, aspirin or naproxen), steroids, or nitrite scavengers (for example ascorbic acid or aminosulphonic acid), or other drugs used for treating gastric ulcers (for example pirenzipine, prostanoids for example 16,16 dimethyl $PGE_2$, or histamine $H_2$-antagonists (for example, cimetidine).

The following examples illustrate the invention. Temperatures are recorded in degrees centigrade.

Example 1

2,4-Bis-(N-methylphenylamino)pyrido[2.3-d]pyrimidine

2,4-Dichloropyrido[2,3-d]pyrimidine (J.A.C.S. (1955), 77, 2256-60) (1.5 g, 0.0075 mol) and N-methylaniline (1.605 g, 0.015 mol) were dissolved in tetrahydrofuran (100 ml) and the mixture was stirred, under reflux, for 16 hours. The solvent was evaporated and the residual oil dissolved in chloroform. The chloroform was washed with aqueous $NaHCO_3$ and water, dried and evaporated to dryness to give a dark-yellow solid. This was treated in ethanol with de-colouring charcoal, filtered and again evaporated to dryness to give a yellow solid. This was treated with diethyl ether, filtered and dried to give the title compound (1.27 g), m.p. 200-205°.

| | $C_{21}H_{19}N_5$. 0.5 $H_2O$ | | |
|---|---|---|---|
| Found | C 71.93, | H.5.70, | N.19.65 |
| Requires | C 71.97, | H 5.75. | N 19.98 |

Example 2

2-[ (2-Methylphenyl)amino]-4-(N-methylphenylamino)pyrido[2,3-d]pyrimidine

(a) 2,4-Dichloropyrido[2,3-d]pyrimidine (5.6 g, 0.028 mol), N-methylaniline (2.29 g, 0.028 mol) and sodium acetate (2.56 g, 0.032 mol) were stirred at room temperature in a mixture of tetrahydrofuran (300 ml) and water (150 ml) for 4 days. The solvent was evaporated under vacuum and the aqueous residue extracted (x2) with chloroform. The combined chloroform extracts were washed with water, dried and evaporated to dryness to give a brown solid. This was treated with petroleum ether, filtered and dried to give 2-chloro-4-(N-methylphenylamino)pyrido-[2,3-d]pyrimidine (5.6 g) which was identified by n.m.r. and mass spectroscopy.

(b) 2-Chloro-4-(N-methylphenylamino)pyrido[2,3-d]-pyrimidine (1.5 g, 0.00554 mol) and o-toluidine (1.17 g, 0.011 mol) were mixed at room temperature and dissolved in absolute ethanol (25 ml). The mixture was heated in a pressure vessel at 140° for 5 hours when a pressure of 110 p.s.i. was noted. After cooling the solvent was removed by evaporation and the residual dark oil partitioned between chloroform and aqueous $NaHCO_3$. The $CHCl_3$ layer was washed with more aqueous $NaHCO_3$ and water, dried and evaporated to dryness to give an oil (2.2 g). This oil was chromatographed on silica gel using chloroform as eluent, followed by chloroform/methanol (50:1). Fractions were monitored by t.l.c. and those containing solely the major product were combined and evaporated to dryness to give a yellow solid. This solid was triturated with petroleum-ether, 40-60 b.p., filtered and dried to give the title compound (0.45 g) as a cream-coloured solid, m.p. 214-217°.

| | $C_{21}H_{19}N_5 0.3H_2O$ | | |
|---|---|---|---|
| Found | C 72.56, | H 5.59, | N 20.26 |
| Requires | C 72.72, | H 5.69, | N 20.19 |

Example 3

2-[ (2-Methyl-4-fluorophenyl)amino]-4-(N-methylphenyl amino)pyrido[2,3-d]pyrimidine hydrochloride

Substituting 4-fluoro-2-methylaniline (1.375 g, 0.011 mol) for o-toluidine and using corresponding molar proportions of the other reagents in Example 2(b) gave, after column chromatography, a sticky solid. This was dissolved in ethanol, ethanolic hydrogen chloride (ethanol saturated with HCl gas) was added and the solution evaporated to dryness. The residue was crystallised from ethanol/diethylether to give the title compound (0.4 g) as its hydrochloride salt, m.p. 227-234°.

| $C_{21}H_{18}FN_5.HCl.0.4\ H_2O$ | | | | |
|---|---|---|---|---|
| Found | C 62,51, | H 4,62, | N 17.41, | Cl 8.87 |
| Requires | C 62.59, | H 4.97, | N 17.37, | Cl 8.79 |

Biological Data.

(A) $H^+K^+$ATPase Activity.

The effects of a single high concentration (100 µM) of a compound of structure (I) on K-stimulated ATPase activity in lyophilised gastric vesicles was determined. Preferred compounds of structure (I) were also tested over a range of concentrations to determine $IC_{50}$ values.

(i) Preparation of lyophilised gastric vesicles (H/K-ATPase).

Lyophilised gastric vesicles were prepared from pig fundic mucosa after the method of Keeling et. al. (Biochem. Pharmacol., 34, 2967, 1985).

(ii) $K^+$-stimulated ATPase activity.

$K^+$-stimulated ATPase activity was determined at 37°C in the presence of the following : 10 mM Pipes/Tris buffer pH 7.0, 2 mM $MgSO_4$, 1 mM KCl, 2 mM $Na_2ATP$ and 3-6 µg protein/ml lyophilised gastric vesicles. After incubation for 30 minutes, the inorganic phosphate hydrolyzed from ATP was determined by the method of Yoda and Hokin (Biochem. Biophys. Res. Commun. 40, 880, 1970).

Compounds of structure (I) were dissolved in dimethylsulphoxide which up to the highest concentration used had no effect on $K^+$-stimulated ATPase activity.

The effect of the highest concentration of each compound of structure (I) on the recovery of a standard amount of inorganic phosphate was also determined.

(iii) Results.

The compounds of the examples had $IC_{50}$ values in the range of from 0.065 to 0.26 µM.

Example A

A tablet for oral administration is prepared by combining

| | Mg/Tablet |
|---|---|
| Compound of structure (I) | 100 |
| lactose | 153 |
| Starch | 33 |
| crospovidone | 12 |
| microcrystalline cellulose | 30 |
| magnesium stearate | 2 |
| | 330 mg |

into a 9 mm tablet.

Example B

An injection for parenteral administration was prepared from the following

| | %w:w |
|---|---|
| Compound of structure (I) | 0,50% (w:v) |
| 1M citric acid | 30% (v:v) |
| sodium hydroxide (qs) | to pH 3.2 |
| water for injection EP | to 100 ml |

The compound of Example 20 was dissolved in the citric acid and the pH slowly adjusted to pH 3.2 with the sodium hydroxide solution. The solution was then made up to 100 ml with water, sterilised by filtration and sealed into appropriately sized ampoules and vials.

**Claims**

1. A compound of structure (I)

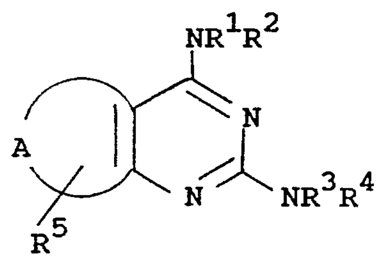

(I)

in which
A is a pyridyl ring;
$R^1$ and $R^2$ are the same, or different and are each hydrogen, $C_{1-4}$alkyl, $-(CH_2)_nAr$ in which n is 0 to 4 and Ar is an optionally substituted phenyl group or $R^1$ and $R^2$ together with the nitrogen atom to which they are attached form a saturated or unsaturated ring optionally containing one or more further heteroatoms;
$R^3$ and $R^4$ are the same or different and are each hydrogen, $C_{1-4}$alkyl, $(CH_2)_nAr^1$ in which n is 0 to 4 and $Ar^1$ is an optionally substituted phenyl group, or $R^3$ and $R^4$ together with the nitrogen atom to which they are attached form a saturated or unsaturated ring optionally containing one or more further heteroatoms; and
$R^5$ is hydrogen or $C_{1-4}$alkyl;
or a pharmaceutically acceptable salt thereof.

2. A compound according to claim 2 in which one of $R^1$ and $R^2$ is $(CH_2)_nAr$ in which n is 0 to 4 and Ar is an optionally substituted phenyl group and the other is $C_{1-4}$alkyl.

3. A compound according to claim 2 in which n is O.

4. A compound according to claim 4 in which one of $R^3$ and $R^4$ is hydrogen and the other is $-(CH_2)_nAr^1$ in which n is 0 to 4 and $Ar^1$ is an optionally substituted phenyl group.

5. A compound according to claim 1 which is
2,4-Bis-(N-methylphenylamino)pyrido[2,3-d]pyrimidine
2-[(2-Methylphenyl)amino]-4-(N-methylphenylamino)pyrido[2,3-d]pyrimidine
2-[(2-Methyl-4-fluorophenyl)amino]-4-(N-methylphenylamino)pyrido[2,3-d]pyrimidine hydrochloride
or a pharmaceutically acceptable salt thereof,

6. A pharmaceutical composition comprising a compound according to any one of claims 1 to 5 and a pharmaceutical carrier.

7. A compound according to any one of claims 1 to 5 for use as a therapeutic agent.

8. A process for the preparation of a compound according to claim 1 which comprises :

(a) reaction of a compound of structure (II)

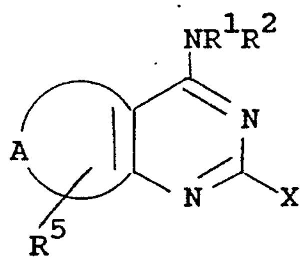

(II)

in which A, $R^1$, $R^2$ and $R^5$ are as described for structure (I) except that where necessary they are in protected form and X is a group displaceable by an amine, with an amine of structure $R^3R^4NH$ in which $R^3$ and $R^4$ are as described for structure (I);

(b) reaction of a compound of structure (III)

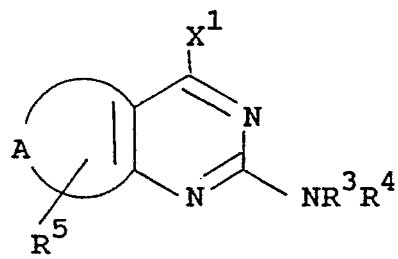

(III)

in which A, $R^3$, $R^4$ and $R^5$ are as described for structure (I) and $X^1$ is a group displaceable by an amine, with an amine of structure $R^1R^2NH$ in which $R^1$ and $R^2$ are as described for structure (I); or (c) and optionally thereafter,

(c) for compounds in which $NR^1R^2$ and $NR^3R^4$ together are the same, reaction of a compound of structure (IV)

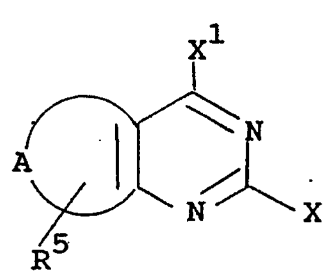

(IV)

in which A and $R^5$ are as described for structure (I), X and $X^1$ are groups displaceable by an amine, with an amine of structure $R^1R^2NH$ or $R^3R^4NH$ in which $R^1$ and $R^4$ are as hereinbefore defined; and optionally thereafter,

° removing any protecting groups;

° forming a pharmaceutically acceptable salt.

9. A compound of structure (II), (III) or (IV).

European Patent Office

# EUROPEAN SEARCH REPORT

Application number

EP 90305635.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US - A - 2 937 284 (HITCHINGS) * Examples 2,4 * | 1,6,7 | C 07 D 471/04<br>A 61 K 31/505<br>//(C 07 D 471/04<br>C 07 D 239:00<br>C 07 D 221:00) |
| X | US - A - 2 926 166 (HITCHINGS) * Examples 21,22; column 1, lines 16-27 * | 1,6-9 | |
| X | GB - A - 755 225 (THE WELLCOME) * Page 1, line 21, formula * | 9 | |
| X | US - A - 3 021 332 (HITCHINGS) * Column 1, lines 11-22; examples 60,63,68,73,74 * | 1-3, 6-9 | |
| X | US - A - 2 749 344 (HITCHINGS) * Column 1, lines 19-21; claim 8, first formula * | 1,6-8 | TECHNICAL FIELDS SEARCHED (Int. Cl.5) |
| X | GB - A - 774 095 (THE WELLCOME) * Examples 54,63; claims 1,2,4 * | 1,6-9 | C 07 D 471/00 |
| X | GB - A - 1 382 487 (DR. KARL THOMAE) * Page 1, formulas I,II; examples 1,5,15,16,20-22,26, 27,30,35,36,38-40,50,51,62, 66,68,70,72,73 * | 1,6-9 | |
| X | US - A - 2 924 599 (OAKES) * Column 1, line 25, formula * | 1 | |
| X | US - A - 3 288 792 | 1,6-8 | |

The present search report has been drawn up for all claims

| Place of search | Date of completion of the search | Examiner |
|---|---|---|
| VIENNA | 24-07-1990 | ONDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number



EP 90305635.6

## DOCUMENTS CONSIDERED TO BE RELEVANT

| Category | Citation of document with indication, where appropriate, of relevant passages | Relevant to claim | CLASSIFICATION OF THE APPLICATION (Int Cl⁵) |
|---|---|---|---|
| | (HITCHINGS) * Column 1, lines 21-37; example 3; column 1, line 71 - column 2, line 41 * -- | | |
| X | US - A - 4 826 528 (MENGEL) * Examples 74,77 * ---- | 1 | |

TECHNICAL FIELDS SEARCHED (Int Cl⁵)

The present search report has been drawn up for all claims

| Place of search | Date of completion of the search | Examiner |
|---|---|---|
| VIENNA | 24-07-1990 | ONDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82